# FASCICULE DE BREVET EUROPEEN

(11) **EP 0 430 837 B1**
(45) Date de publication et mention de la délivrance du brevet: **02.08.1995**
(21) Numéro de dépôt: 90450017.0
(22) Date de dépôt: 29.11.1990
(51) Int. Cl.: A61N 1/05

(54) **Sonde de pace-maker à pole de stimulation auxiliaire**
Herzschrittmachersonde mit zusätzlichem Stimulationspol
Pacemaker probe with auxilliary stimulating pole

(30) Priorité: 30.11.1989 FR 8916075
(43) Date de publication de la demande: 05.06.1991
(73) Titulaire: Bemurat, Marc, F-33850 Leognan (FR)
(72) Inventeur: Bemurat, Marc, F-33850 Leognan (FR)
(74) Mandataire: Thébault, Jean-Louis

(56) Documents cités:
- CH-A- 656 313
- FR-A- 2 537 874
- US-A- 3 437 091

## Description

La présente invention se rapporte aux sondes de stimulation de pace-maker. Une telle sonde est connue par exemple du document CH-A-5 656 313.

Actuellement, sur une sonde de stimulation, seule la tête de la sonde peut recevoir une stimulation électrique. Cette tête de sonde étant introduite dans le boîtier de pace-maker n'est accessible que lorsque la sonde est déconnectée et sortie du boîtier.

Lors du remplacement d'un boîtier de pace-maker, la sonde de stimulation est pratiquement toujours conservée. La manoeuvre consiste à desserrer une vis assurant le contact boîtier-sonde, à sortir la sonde du boîtier, à la réintroduire dans le nouveau boîtier et à resserrer la vis sur la tête de la sonde, enfin, à réenfouir sous la peau l'ensemble sonde et boîtier.

Cette manipulation peut prendre plusieurs dizaines de secondes.

Or, dès que la vis est desserrée, le patient n'est plus stimulé. Cela n'a pas d'inconvénient si le patient garde un rythme cardiaque propre, mais certains patients dits "dépendants" n'ont aucun rythme spontané. Ils sont donc en arrêt cardiaque du moment où la vis de l'ancien boîtier est desserrée jusqu'au moment où la vis du nouveau boîtier assure à nouveau le contact avec la sonde de stimulation.

Chez un patient dépendant, cette manoeuvre est délicate, voire dangereuse.

L'invention vise à palier ce grave inconvénient en proposant une sonde conçue pour assurer la continuité de la stimulation du patient notamment lors du remplacement du boîtier de son pace-maker.

A cet effet, l'invention a pour objet une sonde de pace-maker du type comprenant un cordon souple constitué d'au moins un conducteur électrique spiralé et entouré d'une gaine en matériau isolant, et relié, à une extrémité de la sonde, à une tête amovible de connexion au boîtier du pace-maker et, à l'autre extrémité, à un dispositif à électrode de stimulation, ladite sonde étant caractérisée en ce qu'en un endroit déterminé du cordon ladite gaine présente une solution de continuité rendant accessible de l'extérieur ledit conducteur spiralé, ladite solution de continuité étant susceptible d'être, de manière étanche, isolée de l'extérieur par un moyen mobile.

Dans la présente description on entend par cordon la liaison entre le dispositif à électrode de stimulation et le boîtier du pace-maker, que cette liaison soit constituée d'un cordon unique ou d'un cordon relié par tous moyens d'interconnexion à un raccord dit d'adaptation pourvu d'une tête appropriée, le dispositif de l'invention étant aménagé sur le cordon unique ou sur ledit raccord.

Suivant un mode de réalisation préféré, ladite solution de continuité est constituée par une absence locale au moins partielle de la gaine mettant à nu le conducteur spiralé, cet accès du conducteur étant masqué par un manchon monté coulissant sur le cordon.

Avec un tel dispositif, au moment du changement du boîtier du pace-maker, on fait coulisser ledit manchon, lequel masque l'orifice d'accès au conducteur interne de la sonde durant l'utilisation de celle-ci, de façon à dégager ledit accès pour y insérer une fiche de connexion, telle qu'une pince, en contact avec ledit conducteur. Après branchement d'une stimulation auxiliaire par le canal de cette connexion, on peut débrancher sans aucun risque la tête de sonde du boîtier pour effectuer le changement de ce dernier.

Une fois le nouveau boîtier en place, on débranche la pince de connexion auxiliaire et l'on remet en place le manchon isolant et étanche au dessus dudit accès. On assure ainsi en permanence la stimulation cardiaque du patient tout au long de l'opération.

D'autres caractéristiques et avantages ressortiront de la description qui va suivre de modes de réalisation du dispositif de l'invention, description donnée à titre d'exemple uniquement et en regard du dessin annexé sur lequel :
- Figure 1 est une vue en coupe axiale d'un cordon conforme à l'invention selon un premier mode de réalisation ;
- Figure 2 illustre une variante de réalisation du dispositif de la figure 1, et
- Figure 3 est une vue en coupe axiale d'un second mode de réalisation.

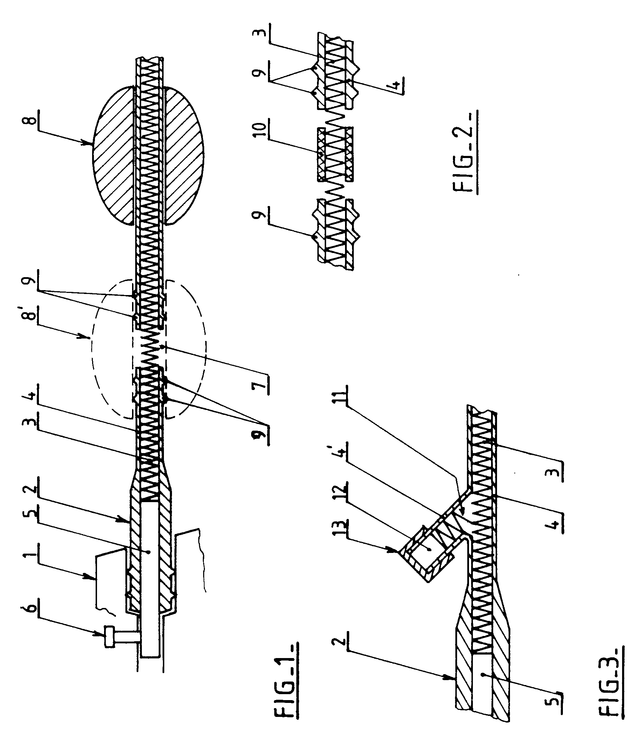

Sur la figure 1 on a représenté schématiquement et partiellement en 1 un boîtier de pace-maker dans lequel est engagée la tête 2 d'une sonde unipolaire de stimulation cardiaque constituée, à la manière connue, d'un conducteur électrique 3 enroulé en spirale à spires sensiblement jointives et entouré d'une gaine 4 en matériau souple isolant électriquement et biocompatible tel que du polyuréthane.

Le conducteur 3 est prolongé, au droit de la tête 2, par une fiche tubulaire femelle 5 destinée à venir en contact électrique avec les circuits internes du boîtier 1 par l'intermédiaire d'une vis 6 de fixation de la tête.

L'autre extrémité du conducteur 3 est reliée au dispositif d'excitation à simple ou double électrode (non représenté).

Conformément à l'invention, en un endroit de la gaine 4, de préférence à proximité de la tête 2, est ménagée une solution de continuité rendant accessible le conducteur interne 3.

Dans le mode de réalisation illustré par la figure 1, cette solution de continuité consiste en une absence locale 7 de gaine, de préférence sur toute la périphérie du cordon, lequel présente ainsi une zone étroite annulaire permettant de connecter provisoirement au conducteur 3 par exemple une pince du type crocodile (non représentée) elle-même reliée à un générateur auxiliaire de stimuli destiné à prendre momentanément le relais du boîtier 1 au moment du débranchement de ce dernier pour le changement de la pile.

En utilisation normale de la sonde, le boîtier 1 étant connecté, la zone découverte 7 de la gaine est masquée et recouverte à l'aide d'un manchon 8 en forme d'olive monté coulissant sur le cordon. Sur la figure 1 on a représenté en pointillés en 8′ la position "normale" du manchon, à cheval sur ladite zone 7.

Le rôle du manchon 8, réalisé par exemple dans le même matériau que la gaine 4, est d'isoler électriquement et de manière étanche la partie dénudée du conducteur 3 du milieu environnant la sonde. En l'absence d'un tel manchon, du fait que le boîtier 1 et la partie proximale du cordon sont disposés sous la peau du patient et donc susceptibles d'être en contact avec un milieu conducteur, il pourrait s'établir des courts-circuits entre le conducteur spiralé 3 et le boîtier qu'il convient absolument d'éviter.

Le manchon 8 doit pouvoir relativement facilement coulisser le long du cordon, aussi, afin d'avoir une étanchéité aussi parfaite que possible, il est avantageusement prévu sur la gaine 4, de part et d'autre de la zone 7, des saillies annulaires 9 légèrement comprimées par le manchon 8 lorsque ce dernier est en position normale (8′) d'isolation du pôle de stimulation auxiliaire constitué dans la zone 7.

Pour avoir un meilleur contact électrique et éviter d'endommager le conducteur spiralé 3, la partie dénudée de ce dernier est de préférence, comme illustré par la figure 2, entouré d'une bague métallique conductrice 10 soudée au conducteur. Il importe en effet de conserver au conducteur 3 sa forme spiralée car le passage interne du conducteur sert à l'introduction d'un mandrin d'insertion de la sonde dans le coeur ou de repositionnement de la sonde.

La figure 3 illustre une autre manière de réaliser une solution de continuité dans la gaine 4 entourant le conducteur 3 permettant un accès à ce dernier. Suivant cet autre mode de réalisation, on constitue une dérivation par exemple en Y à l'aide d'un tronçon 11 de conducteur spiralé dont l'une des extrémités est soudée au conducteur 3 et dont l'autre extrémité est soudée à une fiche de connexion 12, femelle ou mâle, accessible à une fiche de connexion, pince crocodile ou autre, relié à un générateur auxiliaire de stimuli.

Ledit tronçon 11 et au moins une partie de la fiche de connexion auxiliaire 12 sont entourés d'une gaine isolante 4′.

La partie accessible de la fiche 12 est coiffée d'un capuchon 13 l'isolant totalement électriquement et de manière étanche. Le capuchon est par exemple en même matériau que les gaines 4,4′.

Le capuchon 13, engagé à force, est enlevé au moment du changement de la pile du boîtier du pace-maker afin de brancher le générateur auxiliaire juste avant de déconnecter la tête 2.

Le capuchon 13 est remis en place, après reconnexion de la tête 2 dans le boîtier.

Le dispositif de l'invention permet de procéder en toute sécurité, notamment pour les patients dépendants, à la déconnexion de la tête 2 de son boîtier 1 en vue notamment de changer la pile mais aussi de repositionner la sonde dans le coeur à l'aide du mandrin dont la progression à l'intérieur du conducteur spiralé 3 n'est aucunement gênée par l'agencement du pôle de stimulation auxiliaire selon l'invention.

L'invention s'applique également aux sondes bipolaires. Dans ce cas, la solution illustrée en figure 3 est de préférence utilisée, ce qui revient à prévoir côte à côte deux dérivations affectées chacune à l'un des deux conducteurs spiralés de la sonde bipolaire.

Dans le cas où le dispositif de l'invention est aménagé sur un raccord de connexion ou adaptation, cela permet, en particulier, chez des patients appareillés avec un cordon unique, d'interposer entre leur cordon et le boîtier de pace-maker un tel raccord, dont la tête mâle de connexion est identique ou non à la tête de ladite sonde en vue du branchement de cette dernière sur un même boîtier ou sur un boîtier d'un modèle différent. Ainsi il n'est pas nécessaire d'opérer les patients pour changer leur sonde qui pourra de la sorte, grâce au raccord équipé selon l'invention, demeurer sous stimulation à chaque changement ultérieur de boîtier.

Enfin, l'invention n'est évidemment pas limitée aux modes de réalisation représentés et décrits ci-dessus mais en couvre au contraire toutes les variantes, notamment en ce qui concerne les moyens d'accès au conducteur spiralé 3 et les moyens mobiles combinés auxdits moyens d'accès pour autoriser à volonté l'accès audit conducteur ou au contraire son isolation totale électriquement et de manière étanche vis à vis de l'environnement dans lequel est plongée la sonde.

## Revendications

1. Sonde de pace-maker comprenant un cordon souple constitué d'au moins un conducteur électrique spiralé (3) et entouré d'une gaine (4) en matériau isolant, et relié, à une extrémité de la sonde, à une tête (2) amovible de connexion au boîtier (1) du pace-maker et, à l'autre extrémité, à un dispositif à électrode de stimulation, ladite gaine présentant en un endroit déterminé du cordon une solution de continuité (7) rendant accessible de l'extérieur ledit conducteur spiralé (3), ladite sonde étant caractérisée en ce qu'elle comporte un moyen mobile (8), ladite solution de continuité (7) étant susceptible d'être, de manière étanche, isolée de l'extérieur par ledit moyen mobile (8).

2. Sonde suivant la revendication 1, caractérisée en ce que ladite solution de continuité est constituée par une absence locale au moins partielle (7) de la gaine (4) mettant à nu le conducteur spiralé (3), cet accès du conducteur étant masqué par un manchon (8) monté coulissant sur le cordon.

3. Sonde suivant la revendication 2, caractérisée en ce que la gaine (4) est munie, de part et d'autre de ladite zone d'accès (7), de saillies circulaires (9) d'étanchéité susceptibles d'être comprimées par ledit manchon en position (8′) d'isolation de la zone d'accès (7).

4. Sonde suivant la revendication 2 ou 3, caractérisée en ce que la partie dénudée du conducteur spiralé (3) est entourée d'une bague conductrice (10) solidaire dudit conducteur.

5. Sonde suivant la revendication 1, caractérisée en ce que ladite solution de continuité est constituée par une dérivation latérale (11) du conducteur spiralé (3) munie d'une fiche de connexion terminale (12), la dérivation et la fiche étant partiellement entourées d'une gaine isolante (4′) et présentant une partie accessible susceptible d'être coiffée d'un capuchon étanche d'isolation (13).

6. Sonde suivant la revendication 1, caractérisée en ce qu'elle comporte deux conducteurs électriques spiralés, chacun étant muni d'une dérivation latérale suivant la revendication 5.

7. Sonde suivant l'une des revendications 1 à 6, caractérisée en ce que ledit cordon est constitué d'une première partie reliée audit dispositif à électrode de stimulation et d'une seconde partie formant raccord de connexion ou adaptation, ledit endroit déterminé où ladite gaine présente une solution de continuité étant situé sur ledit raccord.

## Claims

1. Pacemaker probe comprising a flexible lead consisting of at least one spirally wound electrical conductor (3) surrounded by a sheath (4) made of insulating material, and connected, at one end of the probe, to a removable head (2) for connection to the casing (1) of the pacemaker and, at the other end, to a stimulation electrode device, the said sheath having, at a given point on the lead, a break in continuity (7) making the said spirally wound conductor (3) accessible from the outside, the said probe being characterised in that it includes a movable means (8), the said break in continuity (7) being capable of being sealingly isolated from the outside by the said movable means (8).

2. Probe according to Claim 1, characterised in that the said break in continuity consists of an at least partial local absence (7) of the sheath (4) baring the spirally wound conductor (3), this access to the conductor being masked by a sleeve (8) slidably mounted on the lead.

3. Probe according to Claim 2, characterised in that the sheath (4) is provided, on each side of the said access zone (7), with circular sealing projections (9) capable of being compressed by the said sleeve in the position (8') of isolation of the access zone (7).

4. Probe according to Claim 2 or 3, characterised in that the bared part of the spirally wound conductor (3) is surrounded by a conductive ring (10) secured to the said conductor.

5. Probe according to Claim 1, characterised in that the said break in continuity consists of a lateral diversion (11) of the spirally wound conductor (3) provided with an end connection contact (12), the diversion and the contact being partially surrounded by an insulating sheath (4') and having an accessible part able to be covered with a sealed insulating cap (13).

6. Probe according to Claim 1, characterised in that it includes two spirally wound electrical conductors, each being provided with a lateral diversion according to Claim 5.

7. Probe according to one of Claims 1 to 6, characterised in that the said lead consists of a first part connected to the said stimulation electrode device and a second part forming a connection or adaptation coupling, the said given point where the said sheath has a break in continuity being situated on the said coupling.

## Patentansprüche

1. Herzschrittmachersonde, umfassend ein weiches Kabel, gebildet aus wenigstens einem spiraligen, elektrischen Leiter (3), der von einer Hülle (4) aus Isoliermaterial umgeben ist, und mit einem Ende der Sonde mit einem auswechselbaren Verbindungskopf (2) zu einem Gehäuse (1) des Schrittmachers sowie mit dem anderen Ende mit einer Anregungselektrodeneinrichtung verbunden, wobei besagtes Gehäuse an einer bestimmten Stelle des Kabels eine Kontinuitätslösung (7) aufweist, die das äußere besagten spiraligen Leiters (3) zugänglich macht, wobei besagte Sonde dadurch gekennzeichnet ist, daß sie ein beweglichen Mittel (8) umfaßt, wobei die Kontinuitätslösung (7) in der Lage ist, in dichter Weise das Äußere durch besagtes bewegliches Mittel (8) zu isolieren.

2. Sonde gemäß Anspruch 1, dadurch gekennzeichnet, daß besagte Kontinuitätslösung durch eine wenigstens partielle, lokale Abwesenheit (7) der Hülle (4) gebildet wird, die den spiraligen Leiter (3) freilegt, wobei dieser Leiterzugang durch eine Hülse (8) isoliert ist, die verschiebbar auf dem Kabel montiert ist.

3. Sonde nach Anspruch 2, dadurch gekennzeichnet, daß die Hülle (4) auf beiden Seiten besagter Zugangszone (7) mit ringförmigen Dichtungsvorsprüngen (9) versehen ist, die geeignet sind, durch besagte Hülse in Isolierposition (8') der Zugangszone (7) komprimiert zu werden.

4. Sonde gemäß Anspruch 2 oder 3, dadurch gekennzeichnet, daß der freigelegte Abschnitt des spiraligen Leiters (3) von einem fest mit besagtem Leiter verbundenen, leitenden Ring (10) umgeben ist.

5. Sonde nach Anspruch 1, dadurch gekennzeichnet, daß besagte Kontinuitätslösung durch eine seitliche Ableitung (11) des spiraligen Leiters (3), versehen mit einem endseitigen Verbindungsstecker (12), gebildet ist, wobei die Ableitung und der Stecker tweilweise von einer isolierenden Hülle (4') ungeben sind und einen zugänglichen Abschnitt aufweisen, der geeignet ist, von einer dichten isolierenden Kappe (13) abgedeckt zu werden.

6. Sonde gemäß Anspruch 1, dadurch gekennzeichnet, daß sie zwei spiralige Leiter umfaßt, von denen jeder mit einer seitlichen Ableitung gemäß Anspruch 5 versehen ist.

7. Sonde gemäß einem der Ansprüche 1 bis 6, dadurch gekennzeichnet, daß besagtes Kabel aus einem ersten, mit besagter Elektrodenstimulationseinrichtung verbundenen Abschnitt und einem zweiten Teil gebildet ist, der das Anschluß- oder Adaptionselement bildet, wobei besagte bestimmte Stelle oder besagte Hülle eine Kontinuitätslösung aufweist, die auf besagtem Element angeordnet ist.
